# EUROPEAN PATENT APPLICATION

(11) **EP 4 674 271 A2**
(43) Date of publication of application: **07.01.2026**
(21) Application number: 25216471.0
(22) Date of filing: 03.02.2016
(51) Int. Cl.: A01P 3/00

(54) **ENCAPSULATION OF HIGH POTENCY ACTIVE AGENTS**

(30) Priority: 03.02.2015 GB 201501793
(62) Divisional of application: 16709486.1
(71) Applicant: Eden Research PLC, Milton Park Abingdon Oxfordshire OX14 4RQ (GB)
(72) Inventor: ABREY, Alexander John, Abingdon, OX14 4RQ (GB); NEWITT, Clive Roland, deceased (GB)
(74) Representative: D Young & Co LLP

(57) **Abstract**

There is described a composition comprising a microparticle component and a highly potent active agent encapsulated in the microparticle.

## Description

### Field of the Invention

The present invention relates to a novel compositions, to methods of their preparation and to uses related thereto.

More particularly the invention relates to compositions comprising microscopic delivery systems, i.e. system for the delivery of active agents, such as biocides, pesticides, such as, fungicides, bactericides, insecticides, etc.; fragrances; flavourings; etc. The invention particularly relates to the delivery of highly potent active agents, i.e. those active agents that are delivered at very low dose rates compared to conventional active agents.

### Background to the Invention

The use of microscopic active agent delivery systems such as those comprising microcapsules, microparticles and liposomes is known.

International Patent application No. WO 2005/104842, Micap plc describes the encapsulation of biocides in fungal cells, for example 15 g of the biocide terbutryn was encapsulated in 180g dried yeast, thus providing a loading of about 0.5% w/w. At page 15 last paragraph, WO '842 describes that a biocidally active compound may be encapsulated in an amount from 1-50 g/100g of product, thus a loading of from 1 to 50% w/w.

International Patent application No. WO 2006/007372 describes a particulate delivery system comprising an extracted yeast cell beta-glucan wall, a payload molecule and a payload trapping molecule. However, the use of a trapping molecule means that generally the loading of the active agent (the payload molecule) in the beta-glucan particle is diminished.

International Patent application No. WO 2005/113128 describes compositions comprising a hollow glucan particles or hollow cell wall particles encapsulating an effective amount of a terpene component which are suitable for preventing and treating infections in plants and animals, including humans, said compositions comprising 1 to 99% by volume terpenes.

It has now been surprisingly found that high potency active agents can be encapsulated in a microparticle with the use of a hollow microparticle.

### Summary of the Invention

Therefore, according to a first aspect of the present invention there is provided a composition comprising a microparticle component and a highly potent active agent encapsulated in the microparticle.

The highly potent active agent may comprise one or more of a biologically active agent, such as, a veterinary active agent, an agrochemical, a fragrance and a flavouring.

In another aspect of the invention the highly potent active agent comprises a veterinary active agent.

In another aspect of the invention the highly potent active agent comprises an agrochemical.

In another aspect of the invention the highly potent active agent comprises a fragrance.

In another aspect of the invention the highly potent active agent comprises a flavouring.

More particularly, the present invention provides a composition wherein the highly potent active agent is an agrochemical. Thus, the present invention provides a composition comprising a microparticle component and an encapsulated highly potent active agent; wherein the loading of the highly potent active agent in the microparticle is from about >1% w/w to about 200% w/w, preferably , preferably from about 1% w/w to about 190% w/w, or from about 1% w/w to about 180% w/w, or from about 1% w/w to about 170% w/w, or from about 1% w/w to about 160% w/w, or from about 1% w/w to about 150% w/w, or from about 1% w/w to about 140% w/w, or from about 1% w/w to about 130% w/w, or from about 1% w/w to about 120% w/w, or from about 1% w/w to about 110% w/w, or from about 1% w/w to about 100% w/w, or from about 1.1% w/w to about 99% w/w, or from about 1.2% w/w to about 98% w/w, or from about 1.3% w/w to about 97% w/w, or from about 1.4% w/w to about 96% w/w, or from about 1.5% w/w to about 95% w/w, or from about 1.6% w/w to about 94% w/w, or from about 1.7% w/w to about 93% w/w, or from about 1.8% w/w to about 920% w/w, or from about 1.9% w/w to about 91% w/w, or from about 2% w/w to about 90% w/w, or from about 5% w/w to about 85% w/w, or from about 10% w/w to about 80% w/w, or from about 15% w/w to about 75% w/w, or from about 20% w/w to about 70% w/w, or from about 25% w/w to about 65% w/w, or from about 30% w/w to about 60% w/w, or from about 35% w/w to about 55% w/w, or from about 40% w/w to about 50% w/w. For the avoidance of doubt, a loading of 1g active agent in 1g of microparticles is deemed to be a loading of 100% w/w.

The active agent may desirably comprise a conventional active agent. By the term "conventional active agent" is meant, for example, one or more of a biologically active agent, such as, a veterinary active agent, an active product and an agrochemical. The term "agrochemical" shall include, for example, a pesticide. A pesticide may include a fungicide, an insecticide, an acaricide, a bactericide, an herbicide, a rodenticide, a growth regulator, etc. Alternatively, the "conventional active agent" may comprise a fragrance, i.e. a perfume, or a flavouring agent.

In a preferred embodiment the highly potent active agent may be a pesticide, e.g. fungicides, insecticides, acaricides, bactericides, herbicides, rodenticides, growth regulators, etc.

For the avoidance of doubt, a highly potent agrochemical shall be deemed to be an agrochemical that is generally utilised in an amount of about 100g per hectare (ha) or less.

In one aspect of the present invention the highly potent agrochemical is an herbicide.

Suitable herbicides include, but shall not be limited to, herbicides selected from the group of sulphonyl ureas, PPO (protoporphyrinogen oxidase) inhibitors aryloxyphenoxypropionates, an hydroxyphenylpyruvate dioxygenase (HPPD) inhibitors and bicyclopyrone.

Examples of sulphonyl ureas include one or more of metsulfuron-methyl, tribenuron-methyl, thifensulfuron-methyl, iodosulfuron, amidosulfuron, rimsulfuron, triflusulfuron-methyl, nicosulfuron and mesosulfuron-methyl.

Examples of PPO inhibitors include one or more of pyraflufen-ethyl, carfentrazone and sulfentrazone.

Examples of aryloxyphenoxypropionates include one or more of propaquizafop, fenoxaprop-ethyl, quiazalofop-P-ethyl, fluazifop-P-butyl and clodinafop-propargyl.

In one aspect of the present invention the herbicide is an hydroxyphenylpyruvate dioxygenase (HPPD) inhibitor.

In another aspect of the present invention the herbicide is bicyclopyrone.

In one aspect of the present invention the highly potent agrochemical is an insecticide.

Suitable insecticides include, but shall not be limited to, pyrethroids.

Examples of pyrethroids include one or more of cypermethrin, alpha-cypermethrin, zeta-cypermethrin, deltamethrin, lambda-cyhalothrin, tau-fluvalinate and pyrethrins (pyrethrin I and pyrethrin II).

In one aspect of the present invention the highly potent agrochemical is a fungicide.

Suitable fungicides include, but shall not be limited to, organic agrochemical fungicides or inorganic mineral fungicides.

An organic agrochemical fungicide may be selected from one or more of chloronitriles, including chlorothalonil, carbamates, including dithiocarbamates such as mancozeb, phtalimides, including captan, sulphonamides, guanidines, quinones, quinolines, thiadiazines, anilides, hydroxyanilides, and phenylamides, imidazolinones, oxazolidinediones, strobilurines, cyanoimidazoles, fluazinam, dinocap, silthiofam, dicarboximides, fludioxonil, organophosphorus, propamocarb HCl, diphenylamine, pyridylamines, sterol biosynthesis inhibitors (SBI) including imidazoles, pyrimidines, hydroxypyrimidines, anilinopyrimidines, triazoles, such as, tebuconazole, spiroxamine, morpholines and piperidines, fenhexamid, hymexazol, zoxamide, diethofencarb, benzimidazoles, pencycuron, quinoxyfen, iprovalicarb, cymoxanil, dimethomorph, phosphonates, triazines, benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, sedaxane and thifluzamide, succinate dehydrogenase inhibiting fungicides, (such as, bixafen, boscalid, carboxin, fluaxapyroxad, fluopyram, isopyrazam, penthiopyrad and sedaxane); and combinations thereof.

Examples of inorganic mineral fungicides include those based on sulfur and/or copper.

Thus, in one aspect of the invention the highly potent active agent is a fragrance.

In another aspect of the invention the highly potent active agent is a flavouring.

It will be understood that the fungal disease to be treated may vary depending, *inter alia* upon the nature of the fungicide. Examples of fungal diseases include, but shall not be limited to stem-base diseases, such as eyespot and cereal ear diseases such as fusarium blight; potato blight, septoria disease of wheat. The composition of the invention may also be suitable for use as a seed dressings, e.g. seedling disease complex in cotton, cereal diseases; such as take-all, loose smut, foot rot, early-season mildew, septoria in wheat, etc.

Useful components of the fragrance include materials of both natural and synthetic origin. They include single compounds and mixtures. Specific examples of such components may be found in the current literature, e.g., in Fenaroli's Handbook of Flavour Ingredients, 1975, CRC Press; Synthetic Food Adjuncts, 1947 by M. B. Jacobs, edited by Van Nostrand; or Perfume and Flavour Chemicals by S. Arctander 1969, Montclair, N.J. (USA). These substances are well known to the person skilled in the art of perfuming and/or aromatizing consumer products, i.e., of imparting an odour to a consumer product that is traditionally perfumed, or of modifying the odour of said consumer product.

It will be understood by the person skilled in the art that the highly potent active agent component of the composition may be "co-encapsulated", that is, encapsulated with a second active agent. The second active agent may be selected from the highly potent active agents described herein or may comprise a terpene component. In one aspect of the invention the second active agent comprises a terpene component.

The choice of terpene as a second active agent may vary and mixtures of terpenes in an appropriate amount may be used. Thus, in one embodiment the terpene component includes one or more terpenes which contain oxygen. Citral, for example citral 95, is an oxygenated C₁₀H₁₆ terpene, C₁₀H₁₆O CAS No. 5392-40-5 (3,7-dimethyl-2,6-octadien- 1-al). A stable suspension of citral can be formed up to about 2500 ppm. Citral can be made into a solution at up to about 500 ppm. A stable suspension of hollow glucan particles incorporating citral of 25 ppt citral can be made.

When a terpene is present, the preferred terpenes are classified as GRAS (Generally Regarded as Safe) by the Environmental Protection Agency in the USA and have been used for many years in the flavour and fragrance industries. The terpenes which are exempted from US regulations and which are listed in EPA regulation 40 C. F.R. Part 152 (incorporated herein by reference in its entirety) are suitable for use in this invention. The building block of the terpenes is the 16 hydrocarbon isoprene (C₅H₈)ₙ.

The term "terpene" as used herein refers not only to terpenes of formula (C₅H₈)ₙ, but also encompasses terpene derivatives, such as terpene aldehydes or terpene polymers. Natural and synthetic terpenes are included, for example monoterpenes, sesquiterpenes, diterpenes, triterpenes, and tetraterpenes. In addition, reference to a single name of a compound will encompass the various isomers of that compound. For example, the term citral includes the cis-isomer citral-a (or geranial) and the trans-isomer, citral-b (or neral).

Particularly suitable terpenes for use in the present invention include those selected from the group consisting of citral, pinene, nerol, β-ionone, geraniol, carvacrol, eugenol, carvone (for example L-carvone), terpeniol, anethole, camphor, menthol, thymol, limonene, nerolidol, farnesol, phytol, carotene (vitamin A₁), squalene, thymol, tocotrienol, perillyl alcohol, borneol, myrcene, simene, carene, terpenene, linalool and mixtures thereof.

The terpenes used in the present invention may have the general structure C₁₀H₁₆.

The terpene component may comprise a terpene selected from the group consisting of one or more of geraniol, thymol, citral, carvone (for example L- carvone), eugenol and β-ionone, or a mixture thereof.

It should be noted that terpenes are also known by the names of the extract or essential oil which contain them, e. g. lemongrass oil (contains citral).

When a terpene component is present in the compositions of the terpene component can comprise a single terpene or a mixture of terpenes as hereinbefore defined. One suitable terpene is citral. A combination of one or more terpenes may also be suitable, such a combination may comprise two or three terpenes.

Certain terpene components which may be suitable include (percentages are w/w of the terpene component; not including the co-encapsulated highly potent active agent):
100% thymol;
100% geraniol;
100% eugenol;
100% citral; or
100% L-carvone.

Other terpene components which may be suitable include thymol; geraniol and thymol; eugenol and thymol; geraniol, eugenol and thymol; eugenol, thymol and citral; geraniol, eugenol, thymol and citral; and geraniol, eugenol, citral, thymol and L-carvone. A terpene component may comprise one or more terpenes selected from the non-limiting group consisting of geraniol, thymol, citral, carvone (for example L-carvone), eugenol and β-ionone. A particular terpene component may comprise a combination of geraniol, thymol and eugenol.

Other terpene components which may be suitable include (percentages are w/w of the terpene component; not including the co-encapsulated highly potent active agent):
100% thymol;
50% geraniol and 50% thymol;
50% eugenol and 50% thymol;
33% geraniol, 33% eugenol and 33% thymol;
40% geraniol, 20% eugenol and 40% thymol;
33% eugenol, 33% thymol and 33% citral;
25% geraniol, 25% eugenol, 25% thymol and 25% citral; and
20% geraniol, 20% eugenol, 20% citral, 20% thymol and 20% L-carvone.

A particular terpene component may comprise a combination of 40% w/w geraniol, 20% w/w eugenol and 40% w/w thymol.

The microparticles of the present invention may comprise a variety of such particles, including, but not limited to, microcapsules, microspheres, liposomes, yeast cell particles, glucan particles, and the like, and mixtures thereof. In order to achieve the high loading of active agent that is an essential element of the present invention, it is desirable that the microparticles as hereinbefore described comprise hollow microparticles. In a particular aspect of the invention the microparticles comprise hollow yeast cell particles or hollow glucan particles.

Microparticles may comprise microcapsules and/or microspheres, usually consisting of substantially spherical particles, for example, 2 mm or less in diameter, usually 500 µm or less in diameter. If the particles are less than 1 µm in diameter they are often referred to as nanocapsules or nanospheres. Microcapsules and microspheres can generally be distinguished from each other by whether a highly potent active agent is formed into a central core surrounded by an encapsulating structure of a matrix material (microcapsules) or whether a highly potent active agent is dispersed throughout the matrix material particle (microspheres). It should be understood that it is within the scope of the present invention to include active agents which are encapsulated within the structure of a matrix material and active agents which are dispersed throughout a matrix material.

A description of methods of making and using microspheres and microcapsules can be found, for example, in International Patent application No. WO 09/013361, which is incorporated herein by reference.

The release of the highly potent active agent from a microcapsule or microsphere is often regulated by the biodegradation of the matrix material. A particularly well known type of microcapsule is liposomes, which can be considered to comprise microcapsules in which the highly potent active agent core is encompassed by a lipid membrane.

Liposomes are artificial lipid vesicles consisting of lipid layers, where the highly potent active agent may be encapsulated inside an aqueous compartment of the liposome, or associated with the surface of the liposome via surface-coupling techniques. Liposomes can be prepared easily and inexpensively on a large scale and under mild conditions.

Other forms of microparticles are yeast cell wall particles or glucan particles. Such particles are readily available, biodegradable and substantially spherical. Yeast cell wall particles and glucan particles are generally about 2-4 µm in diameter. Preparation of extracted yeast cell wall particles is known in the art, and is described, for example, in International Patent application No. WO 2007/063268, which is incorporated herein by reference.

Yeast cell wall particles or glucan particles may be referred to as "whole glucan particles", often referred to as WGPs. Extracted yeast cell wall particles may be referred to as beta-glucan particles.

Such yeast cell wall particles may be in grown form, i.e. may have been harvested from its culture medium, and intact, i.e. not lysed, i.e. the microbe may be alive.

Extracted yeast cell wall particles preferably comprise hollow particles, such as, hollow glucan particles or hollow cell wall particles. The term "hollow glucan particle" as used herein includes any hollow particle comprising glucan as a structural component. Thus, in particular, the term includes hollow yeast cell walls (in purified or crude forms) or hollow glucan particles. The term "cell wall particle" refers to a particle comprising the wall of a cell (in a purified or crude form), wherein glucan is not a structural component. Suitable particles include the cell walls of plant, algal, fungal or bacterial cells. Cell wall particles generally retain the shape of the cell from which they are derived, and thus, like a hollow glucan particle, provide a hollow central cavity suitable for encapsulating the highly potent active agent component. Particularly suitable hollow glucan particles or hollow cell wall particles are fungal cell walls, preferably yeast cell walls.

Yeast cell walls are preparations of yeast cells that retain the three-dimensional structure of the yeast cell from which they are derived. The term hollow particles, such as, hollow glucan particles or hollow yeast cell wall particles is intended to mean glucan microparticles or yeast cell particles wherein intracellular components have been substantially removed. The intracellular components are removed prior to encapsulation of the active encapsulated ingredient, thus enabling the high loading of the highly potent active agent component Removal of the intracellular components may include retaining the desired amount of cellular lipids.

Hollow microparticles, such as, glucan microparticles or yeast cell particles, in which the intracellular components have been substantially removed are known and are commercially available.

Thus, hollow yeast cell particles may suitably be derived from, *inter alia,* Baker's yeast cells (available from Sigma Chemical Corp., St. Louis, MO). Hollow yeast cell particles with desirable properties can also be obtained from Biorigin (Sao Paolo, Brazil) under the trade name Nutricell MOS 55. These particles are a spray dried extract of S. *cerevisiae.*

Glucan particles include, *inter alia,* those known by the trade names SAF-Mannan (SAF Agri, Minneapolis, MN) and Nutrex (Sensient Technologies, Milwaukee, WI). These are hollow glucan particles that are the insoluble waste stream from the yeast extract manufacturing process. During the production of yeast extracts the soluble components of partially autolysed yeast cells are removed and the insoluble residue is a suitable material for loading with a highly potent active agent. These hollow glucan particles comprise approximately 25-35% beta 1,3-glucan w/w.

A key attribute of these hollow microparticles, such as hollow yeast particles and hollow glucan particles, is that they may contain more than 10% lipid w/w and are very effective at absorbing active agents. In addition, as a waste stream product, they are a relatively cheap source of hollow glucan particles. Optionally the hollow microparticles may be treated to remove some or all of any lipid, thus such hollow microparticles may optionally be substantially lipid free.

The term "hollow glucan particle" as used herein includes any hollow particle comprising glucan, e.g. β-glucan, as a structural component. Thus, in particular, the term includes yeast hollow cell walls (in purified or crude forms) or hollow whole glucan particles. Glucan particles are generally 2-4 µm spherical, hollow, porous shells extracted from a yeast, such as Baker's yeast, *Saccharomyces cerevisae.* The surface of the glucan particles is composed primarily of 1,3-β-glucan and the particles. The hollow cavity of the glucan particles allows for efficient absorption and encapsulation of host molecules as active agents. The term "cell wall particles" refers to particles comprising the wall of a cell (in a purified or crude form), wherein glucan is not a structural component or not the main structural component.

The yeast cell wall particles may comprise, for example, Baker's yeast cell walls that are derived from baker's yeast cells and are composed of the insoluble biopolymers β-1, 3-glucan, β-1, 6-glucan, mannan and chitin. They are typically 2-4 µm in diameter microspheres with a shell wall that is only 0.2-0.3 µm thick surrounding an open cavity. This material has considerable liquid holding capacity, typically absorbing 5-25 times its weight in liquid. The shell is sufficiently porous that payloads up to 150,000 Daltons in size can pass through the outer shell and be absorbed into the hollow cavity of the spherical particle. Baker's yeast cell walls have several unique properties, including heat stability (e.g. to 121°C), shear stability, pH stability (e.g. pH 2-12), and at high concentrations they do not build significant viscosity. In addition to its physical properties this composition contains natural and healthy dietary fibres that deliver cardiovascular and immunopotentiation health benefits.

Yeast cell walls are generally prepared from yeast cells by the extraction and purification of the insoluble particulate fraction from the soluble components of the yeast cell. The fungal cell walls can be produced from the insoluble by-product of yeast extract manufacture. Furthermore, the yeast cells can be treated with an aqueous hydroxide solution, without disrupting the yeast cell walls, which digests the protein and intracellular portion of the cell, leaving the yeast cell wall component devoid of significant protein contamination, and having substantially the unaltered cell wall structure of β(1-6) and β(1-3) linked glucans. A more detailed description of whole glucan particles, and the process of preparing them, is described by Jamas et al. in US Patent No. 4,810,646 and in co-pending patent applications US Serial No. 166,929, US Serial No. 297,752 and US Serial No. 297,982. US Patent No. 6,242,594, assigned to Novogen Research Pty Ltd., describes a method of preparing yeast glucan particles by alkali extraction, acid extraction and then extraction with an organic solvent and finally drying. US 5,401,727, assigned to AS Biotech-Mackzymal, discloses the methods of obtaining yeast glucan particles and methods of using them to promote resistance in aquatic animals and as an adjuvant for vaccinations. The teachings of the abovementioned patents and applications are incorporated herein by reference.

Other types of yeast and fungi cells have cell walls that do not contain glucan. The cell walls of such yeast and fungi can be isolated by similar techniques to those mentioned above to obtain cell wall particles.

Additionally, the cells of many plants, algae, bacteria and other micro-organisms also comprise a cell wall. The structure and composition of the cell wall varies between micro-organism, but in general it is a robust and relatively inert structure. It is possible to obtain cell wall particles derived from such cells through conventional techniques, such as those mentioned above in relation to yeast. Thus the term "cell wall particles" shall include yeast cell wall particles and cell wall particles derived from cells of plants, algae, bacteria, etc. as hereinbefore described.

The term "hollow glucan particle" as used herein includes any hollow particle comprising glucan as a structural component. Thus, in particular, the term includes yeast cell walls (in purified or crude forms) or hollow whole glucan particles. The term "cell wall particle" refers to a particle comprising the wall of a cell (in a purified or crude form), wherein glucan is not a structural component.

Suitable particles include the cell walls of plant, algal, fungal or bacterial cells. Cell wall particles generally retain the shape of the cell from which they are derived, and thus, like a hollow glucan particle, provide a hollow central cavity suitable for encapsulating the active agent component.

For this aspect of the present invention it is necessary that the hollow glucan particle or cell wall particle is able to stably encapsulate the highly potent active agent component. In general this means the hollow glucan particle or cell wall particle must be able to maintain its structure during incubation with the highly potent active agent component (generally the highly potent active agent component is at a relatively high concentration), and that active agent component must be able to migrate into the particle. Hollow glucan particles and cell wall particles are generally formed from relatively inert materials and are porous, and thus it can be assumed that, in general, hollow glucan particles and cell wall particles will be able to encapsulate a highly potent active agent component.

Cell wall particles generally retain the shape of the cell from which they are derived, and thus, like a hollow glucan particle, provide a hollow central cavity suitable for encapsulating the highly potent active agent component. Preferred cell wall particles are yeast cell wall particles, e.g. derived from *Saccharomyces cerevisae.*

For this aspect of the present invention it is necessary that the hollow glucan particle or cell wall particle is able to stably encapsulate the highly potent active agent component. In general this means that the hollow glucan particle or hollow cell wall particle must be able to maintain its structure during incubation with the highly potent active agent component (generally the highly potent active agent component is at a relatively high concentration), and that active agent component must be able to migrate into the hollow particle. Hollow glucan particles and hollow cell wall particles are generally formed from relatively inert materials and are porous, and thus it can be assumed that, in general, hollow glucan particles and hollow cell wall particles will be able to encapsulate a highly potent active agent component.

The present invention especially provides a composition as hereinbefore defined wherein the microparticle is a glucan particle or cell wall particle as hereinbefore described. Such glucan particles or cell wall particles may comprise live or intact particles, although as hereinbefore described, in an especially preferred embodiment of the invention the particles comprise hollow glucan particles or hollow yeast cell wall particles, that is, glucan particles or yeast cell particles wherein the intracellular components have been substantially removed.

Yeast cells generally comprise a cell envelope, which is a protective capsule, consisting of three major constituents, the cell wall, the plasma membrane and the periplasmic space. The cell envelope has a major role in controlling the osmotic and permeability properties of the cell. In *S. cerevisiae,* the cell envelope comprises about 15% of the total cell volume. In the embodiment of the present invention providing a hollow microparticle, such as, a hollow glucan particle or hollow yeast cell wall particle, comprising a highly potent active agent component, the highly potent active agent component may be encapsulated in the hollow microparticle. Alternatively, the highly potent active agent component may be held in the cell envelope. It will be understood by the person skilled in the art that it is within the scope of the present invention for a part of the highly potent active agent component to be encapsulated and part to be housed in the cell wall as hereinbefore described.

Particularly suitable hollow glucan particles or cell wall particles are fungal cell walls, preferably yeast cell walls. Yeast cell walls are preparations of yeast cells that retain the three-dimensional structure of the yeast cell from which they are derived. Thus they have a hollow structure which allows the highly potent active agent component to be encapsulated within the yeast cell walls. The yeast walls may suitably be derived from Baker's yeast cells (available from Sigma Chemical Corp., St. Louis, MO). Yeast cell wall particles with desirable properties can also be obtained from Biorigin (Sao Paolo, Brazil) under the trade name Nutricell MOS 55. These particles are a spray dried extract of *S. cerevisiae.*

Alternative particles are those known by the trade names SAF-Mannan (SAF Agri, Minneapolis, MN) and Nutrex (Sensient Technologies, Milwaukee, WI). These are hollow glucan particles that are the insoluble waste stream from the yeast extract manufacturing process. During the production of yeast extracts the soluble components of partially autolysed yeast cells are removed and the insoluble residue is a suitable material for active agent loading. The amount of beta 1,3-glucan in the hollow glucan particles may vary and may be from about 25 to about 90% beta 1,3-glucan w/w. SAF-Mannan hollow glucan particles comprise approximately 25-35% beta 1,3-glucan w/w. A key attribute of these materials is that they contain more than 10% lipid w/w and are very effective at absorbing active agents. In addition, as a waste stream product they are a relatively cheap source of hollow glucan particles.

Alternative hollow glucan particles which have higher purity are those produced by Nutricepts (Nutricepts Inc., Burnsville, MN) and ASA. Biotech. These particles have been alkali extracted, which removes additional intracellular components as well as removes the outer mannoprotein layer of the cell wall yielding a particle of 50-65% w/w glucan.

Higher purity hollow glucan particles are the WGP particles from Biopolymer Engineering. These particles are acid extracted removing additional yeast components yielding a product 75-85% w/w glucan.

Very high purity hollow glucan particles are Adjuvax^{®} from Alpha-beta Technology, Inc. (Worcester, MA) and microparticulate glucan from Novogen (Stamford, CT). These particles are organic solvent extracted which removes residual lipids and so the particles may comprise more than 90% w/w glucan.

In some embodiments a high purity hollow glucan particle or hollow cell wall particle may be required, for example where strict control over possible contaminants is required. In these instances the higher purity particles would be preferred over other less pure products. For other embodiments, the less pure particles would be preferred for economic reasons; those particles have also been found to be more effective at absorbing certain active agents.

The hollow glucan particle or cell wall particle may have a slight lipid content, such as 1 or 2% w/w lipid. A slight lipid content can increase the ability of the particle to encapsulate the highly potent active agent component. The lipid content of the hollow glucan particle or cell wall particle is 5% w/w or greater, or 10% w/w or greater.

Thus, the lipid content of the microparticles, e.g. the hollow glucan particle or hollow cell wall particle may be ≥1% w/w, or ≥2% w/w, or ≥3% w/w, or ≥4% w/w, or ≥5% w/w, or ≥6% w/w, or ≥7% w/w, or ≥8% w/w, or ≥9% w/w, or ≥10% w/w, or ≥15% w/w, or ≥20% w/w, or ≥25%. Thus, the lipid content may be from about 1% to about 25% w/w, or from about 2% to about 20% w/w, or from about 5% to about 15% w/w, e.g. about 10% w/w.

The relative amounts of the highly potent active agent and the terpene may vary depending upon, *inter alia,* the nature and/or potency of the highly potent active agent, the nature of the terpene, etc. Thus, the relative amounts of highly potent active agent to terpene may be such that the co-encapsulated component comprises from about 1% w/w highly potent active agent and about 99% w/w terpene to about 99% w/w highly potent active agent and about 1% w/w terpene.

Thus, the relative amounts of the highly potent active agent and terpene may be from about 1% w/w highly potent active agent and about 99% w/w terpene to about 99% w/w highly potent active agent and about 1% w/w terpene; or from about 10% w/w highly potent active agent and about 90% w/w terpene to about 90% w/w highly potent active agent and about 10% w/w terpene; or from about 20% w/w highly potent active agent and about 80% w/w terpene to about 20% w/w highly potent active agent and about 80% w/w terpene; or from about 30% w/w highly potent active agent and about 70% w/w terpene to about 70% w/w highly potent active agent and about 30% w/w terpene; or from about 40% w/w highly potent active agent and about 60% w/w terpene to about 60% w/w highly potent active agent and about 40% w/w terpene; or from about 50% w/w highly potent active agent and about 50% w/w terpene.

According to a further aspect of the invention there is provided a formulation comprising a composition as hereinbefore described in admixture with a suitable adjuvant diluent or carrier. Thus, said formulation comprises a highly potent active agent encapsulated in a microparticle component; wherein the highly potent active agent in the microparticle is from about 1% w/w to about 100% w/w.

Thus, the microparticle formulations according to this aspect of the invention can contain the biologically active compounds as such or in admixture with one or more agriculturally acceptable auxiliaries, such as carriers, extenders, stabilisers, surface-active agents and colourants.

Thus, the formulation of the present invention can comprise from about 1 ppm to about 25 ppt (25,000 ppm) of the highly potent active agent component (i.e. the component comprising a highly potent active agent encapsulated in a microparticle), based on the total formulation, preferably from about 10 to about 5,000 ppm of the highly potent active agent component, from about 10 to about 5,000 ppm, from about 100 to about 4,000 ppm, from about 200 to about 3,000 ppm, from about 300 to about 2,000 ppm, from about 400 to about 1,500 ppm, from about 500 to about 1,000 ppm. For example, 250, 500, 1000, 2000 ppm thereof. Alternatively, the amount of the highly potent active agent component in the formulation of this aspect of the present invention may comprise from about 0.1% w/w to about 90% w/w of the formulation, based on the total formulation. Therefore, the amount of the highly potent active agent in the formulation may be from about 1% w/w to about 90% w/w, from about 2% w/w to about 90% w/w, from about 3% w/w to about 90% w/w, from about 4% w/w to about 90% w/w, from about 5% w/w to about 90% w/w, from about 6% w/w to about 90% w/w, from about 7% w/w to about 90% w/w, from about 8% w/w to about 90% w/w, from about 9% w/w to about 90% w/w, from about 10% w/w to about 90% w/w, from about 15% w/w to about 90% w/w, from about 20% w/w to about 90% w/w, from about 25% w/w to about 90% w/w, from about 30% w/w to about 90% w/w, from about 35% w/w to about 90% w/w, from about 40% w/w to about 90% w/w, from about 45% w/w to about 90% w/w, from about 50% w/w to about 90% w/w, from about 60% w/w to about 90% w/w, from about 70% w/w to about 90% w/w, from about 80% w/w to about 90% w/w, of the formulation.

In a particular aspect of the present invention there is provided a microparticle delivery system comprising a microparticle, an encapsulated active agent as hereinbefore defined. According to this aspect of the invention the microparticle may comprise yeast cell particles or glucan particles, preferably hollow yeast cell particles or hollow glucan particles; and mixtures thereof.

In another aspect of the invention, when a solvent system is required, e.g. in the formulation of the invention, the solvent system may comprise water.

The microparticle delivery system of this aspect of the present invention may be useful for, *inter alia,* both *in vivo* and *in vitro* delivery of active agents. Therefore, the compositions, formulations and/or the microparticle delivery system of the invention may be useful in the fields of human and/or veterinary medicine and/or agricultural welfare, including, without limitation, the treatment of mammals, e.g. including human, bovine, ovine, porcine, equine, canine and feline species; birds, fish, arthropods and/or plants.

In certain embodiments extracted yeast cell walls comprise less than 90 weight per cent beta-glucan. In certain embodiments the extracted yeast cell walls comprises more than 50 weight per cent chitin. In another embodiment the extracted yeast cell walls further comprise more than 30 weight per cent mannan. In other certain embodiments the extracted yeast cell wall includes more than 1 weight per cent protein. For the avoidance of doubt, extracted yeast cell walls shall be considered to be yeast cells that have had their intracellular components removed, i.e. hollow yeast cells.

The microparticle compositions according to the present invention can contain one or more active agents or combinations of two or more of such agents.

The amount of active agent in the composition may vary, depending upon, *inter alia,* the nature of the highly potent active agent, the intended use of the composition, etc.

Optionally the highly potent active agent component of the composition of the present invention can be associated with a surfactant. The surfactant can be non-ionic, cationic, or anionic. The composition or formylation may optionally comprise from about 0.1 to about 10% w/w surfactant.

Examples of suitable surfactants include sodium lauryl sulphate, polysorbate 20, polysorbate 80, polysorbate 40, polysorbate 60 polyglyceryl ester, polyglyceryl monooleate, decaglyceryl monocaprylate, propylene glycol dicaprilate, triglycerol monostearate, polyoxyethylenesorbitan, monooleate, Tween^{®}, Span^{®} 20, Span^{®} 40, Span^{®} 60, Span^{®} 80, Brig 30 or mixtures thereof. The surfactant acts to hold the terpene component and/or the biologically active component in an emulsion and also assists encapsulation of the terpene component into the microparticle, e.g. hollow glucan particle or hollow cell wall particle.

As hereinbefore described, the formylation of the invention may comprise an active component comprising the encapsulated active agent, i.e. the microparticle/ active agent; with a suitable adjuvant, diluent or carrier. The active component, i.e. the microparticle/ active agent component of the formulation, can comprise from about 1 to about 99% w/w active agent and from about 1 to about 99% w/w microparticle, e.g. hollow glucan particles or hollow cell wall particles. More specifically the formulation can comprise about 10% w/w microparticle and about 90% w/w active agent, about 15% w/w microparticle and about 85% w/w active agent, about 20% w/w microparticle and about 80% w/w active agent, about 25% w/w microparticle and about 75% w/w active agent, about 30% w/w microparticle and about 70% w/w active agent, about 35% w/w microparticle and about 65% w/w active agent, about 40% w/w microparticle and about 60% w/w active agent, about 45% w/w microparticle and about 55% w/w active agent, e.g. about 50% w/w microparticle and about 50% w/w active agent, the remainder comprising a suitable adjuvant, diluent or carrier.

Suitably a formulation of the present invention comprises from about 500 to about 10,000 ppm microparticles, e.g. hollow glucan particles or hollow cell wall particles, where the particles contain an effective amount of a highly potent active agent component as hereinbefore described. Preferably the composition comprises from about 1,000 to about 2,000 ppm microparticles, e.g. hollow glucan particles or hollow cell wall particles, wherein the particles contain a highly potent active agent component.

Concentrations of hollow glucan particles or hollow cell wall particles in the formulation of the invention, for encapsulation of a highly potent active agent, of about 1, 5, 10, 20, 30, 40, 50, 60, 70, 80, 90, 100, 110, 125, 130, 140, 150, 160, 175, 190, 200, 225, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000, 1100, 1250, 1375, 1425, 1500, 1600, 1750, or 2000 ppm, e.g. from about 1ppm to about 1000ppm, can be used as effective concentrations in the compositions, formulations and methods of the current invention. Even higher concentrations (up to 25 ppt, i.e. parts per thousand) can be made and may be useful in the current invention.

Optionally the formulation can comprise other active compounds in addition to those specifically mentioned herein, for example other antimicrobial agents, enzymes, and the like.

The formulations of the invention may also comprise an antioxidant component to reduce oxidation of the microcapsule and/or the highly potent active agents. An example of such an anti-oxidant might be rosemary oil, vitamin C or vitamin E.

The formulations of the present invention may be in the form of a dry powder. The formulations may be provided in combination with an agricultural or food acceptable carrier or excipient in a liquid, solid or gel-like form.

For solid formulations, suitable carriers include agricultural grades of mannitol, lactose, starch, magnesium stearate, sodium saccharin, talc, cellulose, glucose, sucrose, magnesium carbonate, and the like. Suitably the formulation may be formulated in tablet or pellet form.

A pellet, tablet or other solid form of the formulation can preferably also contain a dispersal agent which promotes dispersal of the formulation when placed into a liquid, e.g. water. Suitable dispersal agents include xanthan gum, maltodextrin, alginates, or the like.

Liquid formulations can, for example, be prepared by dispersing the formulation in water, saline, aqueous dextrose, glycerol, ethanol, or the like, to form a solution or suspension. If desired, these formulations can contain minor amounts of non-toxic auxiliary substances such as wetting or emulsifying agents, pH buffering agents (for example, sodium acetate, sorbitan monolaurate, triethanolamine sodium acetate or triethanolamine oleate). The methods of preparing such liquid formulations are known, or will be apparent, to those skilled in this art. A liquid formulation could be prepared by dispersing the formulation in a liquid food or drink material. Additionally a suitable liquid agriculturally acceptable excipient could be used.

Conventionally known carriers, aqueous, powder or oily bases, thickeners, and the like can be used as necessary or desirable.

The present invention further provides a method of delivering a highly potent active agent to a recipient, comprising the steps of:
(i) providing a microparticle component including;
(ii) contacting the microparticle with a highly potent active agent component wherein the highly potent active agent component becomes, at least partially, encapsulated within the microparticle;
(iii) contacting the recipient with the microparticle containing a highly potent active agent component.

It will be understood that the method of this aspect of the invention may comprise delivering a highly potent active agent in the form of a composition or formulation as hereinbefore described.

The recipient may comprise one or more cells or mammals, e.g. including human, bovine, ovine, porcine, equine, canine and feline species; birds, fish, arthropods and/or plants.

The invention further provides a method of treating a body with a highly potent active agent component comprising the step of contacting the cells of an individual with a composition or formulation comprising a microparticle component, a highly potent active agent component, thereby administering to the cells of the individual effective amount of the highly potent active agent.

In the method of treatment of this aspect of the invention, the body may comprise a mammal e.g. bovine, ovine, porcine, equine, canine and feline species. The mammal may especially comprise a human.

Where the highly potent active agent is a pesticide, e.g. an insecticide, the invention may further provide a method of killing a pest, e.g. an arthropod, said method comprising administering an affective amount of a highly potent active agent in the form of a composition or formulation comprising a highly potent active agent component encapsulated in a microparticle component.

The method according to this aspect of the invention may comprise administering the pesticide to a body, plant, etc. Where the highly potent active agent component is a pesticide, e.g. an insecticide, the invention may further provide a method of killing a pest, e.g. an arthropod, said method comprising administering an affective amount of a highly potent active agent in the form of a composition or formulation comprising a highly potent active agent component encapsulated in a microparticle.

It will be understood by the person skilled in the art that this method of the invention may comprise applying the composition of the invention directly to a body as hereinbefore described, or to a plant or to a pest.

According to this aspect of the invention the term "arthropods" includes insects and arachnids, such as, but not limited to, ticks, mites, fleas, mosquitoes, midges, etc.

The amount of the composition of the invention administered will, of course, be dependent on the manner of administration, on the targeted, etc. Suitable compositions are those hereinbefore described.

According to another aspect of the invention there is provided a method of delivering a fragrance, said method comprising administering an affective amount of a highly potent active agent in the form of a composition or formulation comprising a highly potent active agent component encapsulated in a microparticle component as hereinbefore described wherein the highly potent active agent comprises a fragrance.

According to a yet further aspect of the invention there is provided a method of delivering a fragrance, said method comprising administering an affective amount of a highly potent active agent in the form of a composition or formulation comprising a highly potent active agent component encapsulated in a microparticle component according to as hereinbefore described wherein the highly potent active agent comprises a flavouring.

Incorporation of a highly potent active agent component in a microparticle, e.g. a hollow glucan particle or cell wall particle, can reduce the rate of release and/or degradation of the highly potent active agent, thus increasing the duration of action of the highly potent active agent.

Highly potent active agents can be taken up and stably encapsulated within the microparticles, e.g. the hollow glucan particles or hollow cell wall particles. Encapsulation of active agents into such particles can be achieved by incubation of the particles with the highly potent active agent.

The compositions according to the present invention can provide, without limitation, the following advantages:
- maximise active agent encapsulation;
   - minimise unencapsulated active agent;
   - control active agent stability;
   - control active agent release kinetics;
   - creation of a solid form of a liquid active agent to increase the mass and uniformity;
   - simplify handling and application of the highly potent active agent;
   - mask the smell and taste of the highly potent active agent; and
   - inhibit spoilage or decomposition of the composition due to the growth of undesirable mould, yeast, and/or fungus.

The active agent component of the present invention can comprise a single active agent or a mixture of active agents.

The microparticles, active agent components, surfactants, and other components of the compositions of the invention may be readily purchased or synthesised using techniques generally known to synthetic chemists.

The encapsulated active agent may be in liquid form. However, it is within the scope of the present invention for the highly potent active agent to be in solid, e.g. crystalline, form. When the highly potent active agent is in solid form, it may be encapsulated in solid form or, alternatively, may be in solution, suspension, emulsion, etc. Thus, for example, the composition of the invention may optionally include a solvent or a carrier depending upon, *inter alia,* the nature of the highly potent active agent, which may aid in the solubilisation of the highly potent active agent.

The composition of the invention may contain binders and/or lubricants. Fine powders or granules may contain diluting, dispersing and/or surface active agents and can be presented in water or in a syrup.

The composition can conveniently be in a dry state. Non-aqueous solutions or suspensions of the composition are also suitable and may contain suspending agents. Where desirable or necessary, preserving, suspending, thickening, or emulsifying agents can be included.

The composition may also contain buffers, diluents and other suitable additives.

Examples of non-aqueous solvents are propylene glycol, polyethylene glycol, vegetable oils (such as olive oil), and injectable organic esters (such as ethyl oleate). Aqueous carriers include water, alcoholic/aqueous solutions, emulsions, or suspensions, including saline and buffered media. Other vehicles include sodium chloride solution, Ringer's dextrose, dextrose and sodium chloride, lactated Ringer's, or fixed oils.

Preservatives and other additives can also be present such as, for example, antimicrobials, anti-oxidants, chelating agents, and the like.

Conventional carriers, aqueous, powder or oily bases, thickeners, and the like can be used as necessary or desirable.

The present invention also provides a method of making a microparticle delivery system as hereinbefore described, said method comprising the steps of:
providing a microparticle, such as an extracted yeast cell wall comprising beta-glucan, the yeast cell wall defining an internal space;
contacting the microparticle with a preservative amount of a terpene component wherein the terpene component becomes associated with microparticle; and
contacting the microparticle with a highly potent active agent wherein the highly potent active agent becomes associated with microparticle.

In order to achieve the high active agent loading, the microparticle, such as an extracted yeast cell wall comprising beta-glucan, will be a hollow microparticle.

It will be understood by the person skilled in the art that in the method of this aspect of the invention when the composition comprises more than one active agent component associated with the microparticle, each of the highly potent active agent components may be associated with the microparticle separately, simultaneously or sequentially.

The present invention further provides a method of preparing a composition comprising an effective amount of an encapsulated active agent component, said method comprising mixing a microcapsule with an active agent component.

The present invention also provides a method of preparing a pesticidal composition comprising a pesticidally effective amount of an encapsulated pesticidally active agent component said method comprising mixing a microcapsule with a pesticidally active agent component.

More specifically, the method of this aspect of the invention comprises preparing a composition comprising a highly potent active agent component as hereinbefore described wherein the highly potent active agent is in encapsulated form which comprises preparing a microparticle, e.g. a hollow glucan particle or hollow cell wall particle, encapsulating a highly potent active agent, said method comprising the steps of;
a) providing a microparticle, e.g. a hollow glucan particle or cell wall particle;
b) providing a highly potent active agent component;
c) incubating the highly potent active agent component with the microparticle under suitable conditions, e.g. for active agent encapsulation; and
d) recovering the microparticle encapsulated active agent component.

Optionally the above method can further comprise the step of drying the particles encapsulating the highly potent active agent component. Drying may be achieved in a number of ways and mention may be made of freeze drying, fluidised bed drying, drum drying or spray drying, all of which are well known processes.

In step b) of the above method, the highly potent active agent component may be provided as a suspension in a solvent, and optionally in the presence of a surfactant. Suitably the solvent is water. A suitable surfactant is Tween-80 (polyoxyethylenesorbitan monooleate), and preferably the surfactant is present at a concentration of about 0.1 to 10 % by volume of the total reaction mixture, more preferably about 1%. Alternatively the highly potent active agent component may be provided as a true solution in a solvent, e. g. where possible, water.

When a terpene is present being co-encapsulated with the highly potent active agent, a true solution of terpene in water can be obtained by mixing the terpene in water at high shear until a true solution is obtained. Publication No WO 03/020024 provides further details of forming true solutions of terpenes in water.

In step a) of the above method, the microparticle, e.g. the hollow glucan particle or cell wall particle, is suitably provided as a suspension in water or other suitable liquid.

Suitably the suspension comprises approximately 1 to 1000 mg particles per ml, preferably 200 to 400 mg/ml. Alternatively the particles may be provided as a dry powder and added to the terpene-surfactant suspension.

Alternatively the particles are provided in sufficient liquid to minimally hydrate the particles, but not in significant excess. The term "hydrodynamic volume" (HV) is used to describe the volume of liquid required to minimally hydrate the particles. Thus suitably the particles are provided with a volume ranging from the HV and a volume of 1.5 times the HV (1.5HV). This makes the subsequent drying step more efficient. Also, where a low volume of liquid is used (i.e. around HV to 1.5HV), it is also possible to extrude the finished product into pellet or noodle form, which is convenient for fluidised bed drying.

It has been found that the terpene component can become encapsulated by the hollow glucan particle or cell wall particle at room temperature. The rate of encapsulation is, however, increased at 37°C but the temperature should be kept below the boiling point or denaturing temperature of any component of the composition. Suitable conditions for step c) of the above method are therefore atmospheric pressure at a temperature of 20 to 37°C. Optimisation of the conditions for a particular encapsulation reaction will be a matter of routine experimentation.

Optionally the above method can further comprise the step of drying the particles encapsulating the highly potent active agent component. Drying may be achieved in a number of ways and mention may be made of freeze drying, fluidised bed drying, drum drying or spray drying, all of which are well known processes.

Therefore, according to a yet further aspect of the present invention there is provided the use of a highly potent active agent component in the manufacture of a microparticle composition as hereinbefore described.

According to this aspect of the invention the microparticles are preferably glucan particles or yeast particles, e.g. hollow glucan particles or hollow yeast particles as hereinbefore described.

The invention will now be described in further detail in the following numbered paragraphs:
1. A composition comprising a microparticle component and a highly potent active agent encapsulated in the microparticle.
2. A composition according to numbered paragraph 1 wherein the highly potent active agent comprises one or more of a biologically active agent, such as, a veterinary active agent, an agrochemical, a fragrance and a flavouring.
3. A composition according to numbered paragraphs 1 or 2 wherein the highly potent active agent is an agrochemical.
4. A composition according to any one of the preceding numbered paragraphs wherein the loading of the in the microparticle is from about 1% w/w to about 200% w/w.
5. A composition according to any one of the preceding numbered paragraphs wherein the highly potent active agent comprises a pesticide.
6. A composition according to numbered paragraph 5 wherein the pesticide is selected from one or more of a fungicide, an insecticide, an acaricide, a bactericide, an herbicide, an rodenticide, a growth regulator, etc.
7. A composition according to numbered paragraphs 5 or 6 wherein the pesticide is an herbicide.
8. A composition according to numbered paragraphs 6 or 7 wherein the herbicide is selected from the group of sulphonyl ureas, PPO (protoporphyrinogen oxidase) inhibitors and aryloxyphenoxypropionates.
9. A composition according to numbered paragraph 8 wherein the herbicide is a sulphonyl urea.
10. A composition according to numbered paragraphs 8 or 9 wherein the sulphonyl urea is one or more of metsulfuron-methyl, tribenuron-methyl, thifensulfuron-methyl, iodosulfuron, amidosulfuron, rimsulfuron, triflusulfuron-methyl, nicosulfuron and mesosulfuron-methyl.
11. A composition according to numbered paragraphs 8 to 10 wherein the sulphonyl urea is metsulfuron-methyl.
12. A composition according to numbered paragraphs 8 to 10 wherein the sulphonyl urea is tribenuron-methyl.
13. A composition according to numbered paragraphs 8 to 10 wherein the sulphonyl urea is thifensulfuron-methyl.
14. A composition according to numbered paragraphs 8 to 10 wherein the sulphonyl urea is iodosulfuron.
15. A composition according to numbered paragraphs 8 to 10 wherein the sulphonyl urea is amidosulfuron.
16. A composition according to numbered paragraphs 8 to 10 wherein the sulphonyl urea is rimsulfuron.
17. A composition according to numbered paragraphs 8 to 10 wherein the sulphonyl urea is triflusulfuron-methyl.
18. A composition according to numbered paragraphs 8 to 10 wherein the sulphonyl urea is nicosulfuron.
19. A composition according to numbered paragraphs 8 to 10 wherein the sulphonyl urea is mesosulfuron-methyl.
20. A composition according to numbered paragraph 8 wherein the herbicide is a PPO (protoporphyrinogen oxidase) inhibitor.
21. A composition according to numbered paragraph 20 wherein the PPO inhibitor is one or more of pyraflufen-ethyl, carfentrazone and sulfentrazone.
22. A composition according to numbered paragraph 8 wherein the herbicide is an aryloxyphenoxypropionate.
23. A composition according to numbered paragraph 22 wherein the aryloxyphenoxypropionate is one or more of propaquizafop, fenoxaprop-ethyl, quiazalofop-P-ethyl, fluazifop-P-butyl and clodinafop-propargyl.
24. A composition according to numbered paragraph 8 wherein the herbicide is an hydroxyphenylpyruvate dioxygenase (HPPD) inhibitor.
25. A composition according to numbered paragraph 8 wherein the herbicide is bicyclopyrone.
26. A composition according to numbered paragraphs 5 or 6 wherein the pesticide is an insecticide.
27. A composition according to numbered paragraph 26 wherein the insecticide is a pyrethroid.
28. A composition according to numbered paragraph 27 wherein the pyrethroid is one or more of cypermethrin, alpha-cypermethrin, zeta-cypermethrin, deltamethrin, lambda-cyhalothrin, tau-fluvalinate and pyrethrins (pyrethrin I and pyrethrin II).
29. A composition according to numbered paragraphs 5 or 6 wherein the pesticide is a fungicide.
30. A composition according to numbered paragraph 29 wherein the fungicide is an organic agrochemical fungicide.
31 A composition according to numbered paragraph 30 wherein the organic agrochemical fungicide is selected from one or more of chloronitriles, including chlorothalonil, carbamates, including dithiocarbamates such as mancozeb, phtalimides, including captan, sulphonamides, guanidines, quinones, quinolines, thiadiazines, anilides, hydroxyanilides, and phenylamides, imidazolinones, oxazolidinediones, strobilurines, cyanoimidazoles, fluazinam, dinocap, silthiofam, dicarboximides, fludioxonil, organophosphorus, propamocarb HCl, diphenylamine, pyridylamines, sterol biosynthesis inhibitors (SBI) including imidazoles, pyrimidines, hydroxypyrimidines, anilinopyrimidines, triazoles, spiroxamine, morpholines and piperidines, fenhexamid, hymexazol, zoxamide, diethofencarb, benzimidazoles, pencycuron, quinoxyfen, iprovalicarb, cymoxanil, dimethomorph, phosphonates, triazines, benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, sedaxane and thifluzamide, succinate dehydrogenase inhibiting fungicides, such as, (bixafen, boscalid, carboxin, fluaxapyroxad, fluopyram, isopyrazam, penthiopyrad and sedaxane); and combinations thereof.
32. A composition according to numbered paragraph 29 wherein the fungicide is an inorganic mineral fungicide.
33. A composition according to numbered paragraph 32 wherein the inorganic mineral fungicide is based on sulfur.
34 A composition according to numbered paragraph 32 wherein the inorganic mineral fungicide is based on copper.
35. A composition according to numbered paragraphs 1 or 2 wherein the highly potent active agent is a fragrance.
36. A composition according to numbered paragraphs 1 or 2 wherein the highly potent active agent is a flavouring.
37. A composition according to any one of the preceding numbered paragraphs wherein the highly potent active agent component of the composition is "co-encapsulated" with a second active agent.
38. A composition according to numbered paragraph 37 wherein the second active agent is a terpene component.
39. A composition according to numbered paragraph 38 wherein the terpene component is selected from the group consisting of citral, pinene, nerol, β-ionone, geraniol, carvacrol, eugenol, carvone (for example L-carvone), terpeniol, anethole, camphor, menthol, thymol, limonene, nerolidol, farnesol, phytol, carotene (vitamin A₁), squalene, thymol, tocotrienol, perillyl alcohol, borneol, myrcene, simene, carene, terpenene, linalool and mixtures thereof.
40. A composition according to numbered paragraphs 38 or 39 wherein the terpene component is selected from the group consisting of one or more of geraniol, thymol, citral, carvone (for example L- carvone), eugenol and β-ionone, or a mixture thereof.
41. A composition according to any one of numbered paragraphs 38 to 40 wherein the terpene component comprises a single terpene.
42. A composition according to any one of numbered paragraphs 38 to 40 wherein the terpene component comprises a combination of two terpenes.
43. A composition according to any one of numbered paragraphs 38 to 40 wherein the terpene component comprises a combination of three terpenes.
44. A composition according to any one of numbered paragraphs 38 to 40 wherein the terpene component is selected from:
   100% thymol;
   100% geraniol;
   100% eugenol;
   100% citral; or
   100% L-carvone.
45. A composition according to any one of numbered paragraphs 38 to 40 wherein the terpene component is selected from:
   100% thymol;
   50% geraniol and 50% thymol;
   50% eugenol and 50% thymol;
   33% geraniol, 33% eugenol and 33% thymol;
   40% geraniol, 20% eugenol and 40% thymol;
   33% eugenol, 33% thymol and 33% citral;
   25% geraniol, 25% eugenol, 25% thymol and 25% citral; and
   20% geraniol, 20% eugenol, 20% citral, 20% thymol and 20% L-carvone.
46. A composition according to numbered paragraphs 38 to 40 wherein the terpene component comprises a combination of geraniol, thymol and eugenol.
47. A composition according to numbered paragraph 46 wherein the terpene component comprises a combination of 40% w/w geraniol, 20% w/w eugenol and 40% w/w thymol.
48. A composition according to any one of numbered paragraphs 38 to 47 wherein the relative amounts of the highly potent active agent and terpene may be from about 1% w/w highly potent active agent and about 99% w/w terpene to about 99% w/w highly potent active agent and about 1% w/w terpene.
49. A composition according to any one of the preceding numbered paragraphs wherein the microparticles about 2-4 µm in diameter.
50. A composition according to any one of the preceding numbered paragraphs wherein the microparticles comprise hollow fungal cell particles or hollow glucan particles.
51. A composition according to numbered paragraph 50 wherein the microparticles are hollow glucan particles.
52. A composition according to numbered paragraph 50 wherein the microparticles are hollow cell wall particles.
53. A composition according to numbered paragraph 50 or wherein the microparticles are hollow fungal cell walls.
54. A composition according to numbered paragraph 53 or wherein the microparticles are hollow yeast cell walls.
55. A composition according to numbered paragraph 54 or wherein the hollow yeast cell particles are derived from Baker's yeast cells.
56. A composition according to numbered paragraph 55 or wherein the yeast cell particles are a spray dried extract of *S. cerevisiae.*
57. A composition according to numbered paragraph 53 wherein the hollow glucan particles are SAF-Mannan glucan particles.
58. A composition according to numbered paragraph 53 wherein the hollow glucan particles are Nutrex hollow glucan particles.
59. A composition according to any one of numbered paragraphs 51, 57 or 58 wherein the cell wall particle comprises more than 90% w/w glucan.
60. A composition according to numbered paragraph 59 wherein the cell wall particle comprises 75-85% w/w glucan.
61. A composition according to numbered paragraph 60 wherein the cell wall particle comprises 50-65% w/w glucan.
62. A composition according to numbered paragraphs 51, 57 or 58 wherein the amount of beta 1,3-glucan in the hollow glucan particles is from about 25 to about 90% w/w.
63. A composition according to numbered paragraph 62 wherein the hollow glucan particles comprise approximately 25-35% beta 1,3-glucan w/w.
64. A composition according to any one of the preceding numbered paragraphs wherein the microparticles are substantially lipid free.
65. A composition according to any one of numbered paragraphs 1 to 63 wherein the microparticles contain ≥1% w/w lipid.
66. A composition according to numbered paragraph 65 wherein the microparticles contain 1 or 2% w/w lipid.
67. A composition according to numbered paragraphs 65 or 66 wherein the microparticles contain more than 5% lipid w/w.
68. A composition according to numbered paragraph 67 wherein the microparticles contain more than 10% lipid w/w.
69. A composition according to any one of the preceding numbered paragraphs wherein the composition includes a surfactant.
70. A composition or formulation any one of the preceding numbered paragraphs in the form of a dry powder.
71. A formulation comprising a composition according to any one of the preceding numbered paragraphs in admixture with a suitable adjuvant diluent or carrier.
72. A formulation according to numbered paragraph 71 comprising a highly potent active agent encapsulated in a microparticle component; wherein the loading of the highly potent active agent in the microparticle is from about 1% w/w to about 100% w/w.
73. A formulation according to numbered paragraphs 71 or 72 wherein the formulation includes a solvent.
74. A formulation according to numbered paragraph 73 wherein the solvent comprises water.
75. A formulation according to numbered paragraph 73 wherein the formulation comprises a non-aqueous solvent.
76. A formulation according to any one of numbered paragraphs 71 to 75 comprising about 10% w/w microparticle and about 90% w/w highly potent active agent.
77. A formulation according to any one of numbered paragraphs 71 to 75 comprising from about 1 to about 10,000 ppm microparticles wherein the particles contain a highly potent active agent component.
78. A formulation according to numbered paragraph 77 comprising from about 500ppm to about 1000ppm.
79. A formulation according to any one of numbered paragraphs 71 to 78 wherein the formulation comprises binders and/or lubricants.
80. A formulation according to any one of numbered paragraphs 71 to 79 wherein the formulation comprises a preservative.
81. A microparticle delivery system comprising a microparticle component and a highly potent active agent encapsulated in the microparticle component.
82. A method of delivering a highly potent active agent to a recipient, comprising the steps of:
   (i) providing a microparticle component including;
   (ii) contacting the microparticle with an highly potent active agent component wherein the highly potent active agent component becomes, at least partially, encapsulated within the microparticle;
   (iii) contacting the recipient with the microparticle containing a highly potent active agent component.
83. A method according to numbered paragraph 82 wherein the recipient is a mammal.
84. A method according to numbered paragraph 82 wherein the recipient is an arthropod.
85. A method of killing a pest, said method comprising administering an affective amount of a highly potent active agent in the form of a composition or formulation comprising a highly potent active agent component encapsulated in a microparticle component according to numbered paragraph 5 wherein the highly potent active agent comprises a pesticide.
86. A method according to numbered paragraph 85 which comprises administering the pesticide to a body.
87. A method according to numbered paragraph 85 which comprises administering the pesticide to a plant.
88. A method of killing a pest according to any one of numbered paragraphs 85 to 87 wherein the pesticide is an herbicide.
89. A method according to numbered paragraph 88 wherein the herbicide is selected from the group of sulphonyl ureas, PPO (protoporphyrinogen oxidase) inhibitors and aryloxyphenoxypropionates.
90. A method according to numbered paragraph 89 wherein the herbicide is a sulphonyl urea.
91. A method according to numbered paragraphs 89 or 90 wherein the sulphonyl urea is one or more of metsulfuron-methyl, tribenuron-methyl, thifensulfuron-methyl, iodosulfuron, amidosulfuron, rimsulfuron, triflusulfuron-methyl, nicosulfuron and mesosulfuron-methyl.
92. A method according to numbered paragraph 89 wherein the herbicide is a PPO (protoporphyrinogen oxidase) inhibitor.
93. A method according to numbered paragraph 92 wherein the PPO inhibitor is one or more of pyraflufen-ethyl, carfentrazone and sulfentrazone.
94. A method according to numbered paragraph 89 wherein the herbicide is an aryloxyphenoxypropionate.
95. A method according to numbered paragraphs 89 or 94 wherein the aryloxyphenoxypropionate is one or more of propaquizafop, fenoxaprop-ethyl, quiazalofop-P-ethyl, fluazifop-P-butyl and clodinafop-propargyl.
96. A method of killing a pest according to any one of numbered paragraphs 85 to 87 wherein the pesticide is an insecticide.
97. A method according to numbered paragraph 96 wherein the insecticide is a pyrethroid.
98. A method according to numbered paragraphs 96 or 97 wherein the pyrethroid is one or more of cypermethrin, alpha-cypermethrin, zeta-cypermethrin, deltamethrin, lambda-cyhalothrin, tau-fluvalinate and pyrethrins (pyrethrin I and pyrethrin II).
99. A method of killing a pest according to any one of numbered paragraphs 96 to 98 wherein the pest is an insect.
100. A method of killing a pest according to numbered paragraph 99 wherein the pest is a flea, mosquito, midge, etc.
101. A method of killing a pest according to any one of numbered paragraphs 96 to 98 wherein the pest is an arachnid.
102. A method of killing a pest according to numbered paragraph 101 wherein the pest is a tick or mite.
103. A method of killing a pest according to any one of numbered paragraphs 85 to 102 wherein the highly potent active agent is co-encapsulated with one or more terpenes.
104. A method of killing a pest according to numbered paragraph 103 wherein the one or more terpenes comprises a combination of geraniol, thymol and eugenol.
105. A method of killing a pest according to numbered paragraph 104 the terpene component comprises a combination of 40% w/w geraniol, 20% w/w eugenol and 40% w/w thymol.
106. A method of killing a pest according to any one of numbered paragraphs 85 to 87 wherein the pesticide is a fungicide.
107. A method of killing a pest according to numbered paragraph 106 wherein the fungicide is an organic agrochemical fungicide.
108. A method of killing a pest according to numbered paragraph 107 the organic agrochemical fungicide is selected from one or more of chloronitriles, including chlorothalonil, carbamates, including dithiocarbamates such as mancozeb, phtalimides, including captan, sulphonamides, guanidines, quinones, quinolines, thiadiazines, anilides, hydroxyanilides, and phenylamides, imidazolinones, oxazolidinediones, strobilurines, cyanoimidazoles, fluazinam, dinocap, silthiofam, dicarboximides, fludioxonil, organophosphorus, propamocarb HCl, diphenylamine, pyridylamines, sterol biosynthesis inhibitors (SBI) including imidazoles, pyrimidines, hydroxypyrimidines, anilinopyrimidines, triazoles, spiroxamine, morpholines and piperidines, fenhexamid, hymexazol, zoxamide, diethofencarb, benzimidazoles, pencycuron, quinoxyfen, iprovalicarb, cymoxanil, dimethomorph, phosphonates, triazines, benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, sedaxane and thifluzamide, succinate dehydrogenase inhibiting fungicides, such as, (bixafen, boscalid, carboxin, fluaxapyroxad, fluopyram, isopyrazam, penthiopyrad and sedaxane); and combinations thereof.
109. A method of killing a pest according to numbered paragraph 106 wherein the fungicide is an inorganic mineral fungicide.
110. A method of killing a pest according to numbered paragraph 109 wherein the inorganic mineral fungicide is based on sulfur.
111. A method of killing a pest according to numbered paragraph 109 wherein the inorganic mineral fungicide is based on copper.
112. A method of delivering a fragrance, said method comprising administering an affective amount of a highly potent active agent in the form of a composition or formulation comprising a highly potent active agent component encapsulated in a microparticle component according to numbered paragraph 35 wherein the highly potent active agent comprises a fragrance.
113. A method of delivering a fragrance, said method comprising administering an affective amount of a highly potent active agent in the form of a composition or formulation comprising a highly potent active agent component encapsulated in a microparticle component according to numbered paragraph 36 wherein the highly potent active agent comprises a flavouring.
114. A method of making a microparticle delivery system according to numbered paragraph 81, said method comprising the steps of:
   providing a microparticle, such as an extracted yeast cell wall comprising beta-glucan, the yeast cell wall defining an internal space;
   contacting the microparticle with a highly potent active agent component wherein the highly potent active agent component becomes associated with microparticle.
115. The use of a highly potent active agent component in the manufacture of a microparticle composition according to any one of numbered paragraphs 1 to 70.
116. The use according to numbered paragraph 115 wherein the microparticles are glucan particles or yeast particles.
117. A composition, formulation, method, delivery system or use substantially as hereinbefore described with reference to the accompanying description.

## Claims

1. A composition comprising a microparticle component and a highly potent active agent encapsulated in the microparticle, the microparticle component comprising hollow fungal cell particles or hollow glucan particles,
wherein the highly potent active agent is a pesticide, and
wherein the loading of the highly potent active agent in the microparticle component is from 1% w/w to 200% w/w.

2. A composition according to claim 1 wherein the pesticide is selected from one or more of a fungicide, an insecticide, an acaricide, a bactericide, an herbicide, a rodenticide, and a growth regulator.

3. A composition according to claim 2 wherein the pesticide is an herbicide selected from the group of sulphonyl ureas, PPO (protoporphyrinogen oxidase) inhibitors and aryloxyphenoxypropionates.

4. A composition according to claim 3 wherein the herbicide is a sulphonyl urea selected from one or more of metsulfuron-methyl, tribenuron-methyl, thifensulfuron-methyl, iodosulfuron, amidosulfuron, rimsulfuron, triflusulfuron-methyl, nicosulfuron and mesosulfuron-methyl.

5. A composition according to claim 3 wherein the herbicide is a PPO (protoporphyrinogen oxidase) inhibitor selected from one or more of pyraflufen-ethyl, carfentrazone and sulfentrazone.

6. A composition according to claim 3 wherein the herbicide is an aryloxyphenoxypropionate selected from one or more of propaquizafop, fenoxaprop-ethyl, quiazalofop-P-ethyl, fluazifop-P-butyl and clodinafop-propargyl.

7. A composition according to claim 2 wherein the pesticide is an insecticide selected from one or more of cypermethrin, alpha-cypermethrin, zeta-cypermethrin, deltamethrin, lambda-cyhalothrin, tau-fluvalinate and pyrethrins.

8. A composition according to claim 2 wherein the pesticide is a fungicide selected from one or more of chloronitriles, including chlorothalonil, carbamates, including dithiocarbamates such as mancozeb, phtalimides, including captan, sulphonamides, guanidines, quinones, quinolines, thiadiazines, anilides, hydroxyanilides, and phenylamides, imidazolinones, oxazolidinediones, strobilurines, cyanoimidazoles, fluazinam, dinocap, silthiofam, dicarboximides, fludioxonil, organophosphorus, propamocarb HCl, diphenylamine, pyridylamines, sterol biosynthesis inhibitors (SBI) including imidazoles, pyrimidines, hydroxypyrimidines, anilinopyrimidines, triazoles, spiroxamine, morpholines and piperidines, fenhexamid, hymexazol, zoxamide, diethofencarb, benzimidazoles, pencycuron, quinoxyfen, iprovalicarb, cymoxanil, dimethomorph, phosphonates, triazines, benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, sedaxane and thifluzamide, succinate dehydrogenase inhibiting fungicides, such as, (bixafen, boscalid, carboxin, fluaxapyroxad, fluopyram, isopyrazam, penthiopyrad and sedaxane); and combinations thereof.

9. A composition according to any one of the preceding claims wherein the highly potent active agent component of the composition is "co-encapsulated" with a terpene component.

10. A composition according to claim 9 wherein the terpene component is selected from the group consisting of citral, pinene, nerol, β-ionone, geraniol, carvacrol, eugenol, carvone, terpeniol, anethole, camphor, menthol, thymol, limonene, nerolidol, farnesol, phytol, carotene, squalene, thymol, tocotrienol, perillyl alcohol, borneol, myrcene, simene, carene, terpenene, linalool and mixtures thereof.

11. A composition according to claim 9 or 10 wherein the relative amounts of the highly potent active agent and terpene may be from about 1% w/w highly potent active agent and about 99% w/w terpene to about 99% w/w highly potent active agent and about 1% w/w terpene.

12. A composition according to claim 1 wherein the microparticles are hollow yeast cell walls.

13. A composition according to any one of claims 1 to 12 wherein the microparticles contain more than 5% lipid w/w.

14. A formulation comprising a composition according to any one of the preceding claims in admixture with a suitable carrier, wherein the carrier is water.

15. A method of killing a pest, said method comprising administering an effective amount of a highly potent active agent in the form of a composition according to any one of claims 1 to 13, or formulation according to claim 14.
